# EUROPEAN PATENT SPECIFICATION

(11) **EP 3 765 033 B1**
(45) Date of publication and mention of the grant of the patent: **27.04.2022**
(21) Application number: 19714248.2
(22) Date of filing: 08.03.2019
(51) Int. Cl.: A61K 31/765, A61P 35/00

(54) **POLYHYDROXYALKANOATES FOR USE IN THE PREVENTION OF COLORECTAL CANCER**
POLYHYDROXYALKANOATE ZUR VERWENDUNG BEI DER BEHANDLUNG VON KOLOREKTALKARZINOM
POLYHYDROXYALCANOATES DESTINÉS À ÊTRE UTILISÉS DANS LA PRÉVENTION DU CANCER COLORECTAL

(30) Priority: 12.03.2018 IT 201800003441
(43) Date of publication of application: 20.01.2021
(73) Proprietor: Bio-On S.p.A., 40016 San Giorgio Di Piano (BO) (IT)
(72) Inventor: COMES FRANCHINI, Mauro, 40016 San Giorgio Di Piano (Bologna) (IT); SAETTONE, Paolo, 40024 Castel San Pietro Terme (Bologna) (IT); FERNANDEZ, Javier, 33003 Oviedo, Asturias (ES); VILLAR, Claudio Jesús, 33003 Oviedo, Asturias (ES); LOMBÓ, Felipe, 33003 Oviedo, Asturias (ES)
(74) Representative: Bottero, Carlo
(86) International application number: PCT/IB2019/051891
(87) International publication number: WO 2019/175725

(56) References cited:
- WO-A1-2014/106026
- IONUT-CRISTIAN RADU ET AL: "Poly(HydroxyButyrate-co-HydroxyValerate) (PHBHV) Nanocarriers for Silymarin Release as Adjuvant Therapy in Colo-rectal Cancer", FRONTIERS IN PHARMACOLOGY, vol. 8, 2 August 2017 (2017-08-02), XP055522298, CH ISSN: 1663-9812, DOI: 10.3389/fphar.2017.00508
- J THULESEN: "Glucagon-like peptide 2 (GLP-2) accelerates the growth of colonic neoplasms in mice", GUT, vol. 53, no. 8, 1 August 2004 (2004-08-01) , pages 1145-1150, XP055522274, UK ISSN: 0017-5749, DOI: 10.1136/gut.2003.035212

## Description

The present invention relates to polyhydroxyalkanoates (PHAs) for use in the prevention of colorectal cancer (CRC).

Colorectal cancer (CRC), also known as bowel cancer or colon cancer, is a type of cancer which develops in colon or rectum, which are parts of the large intestine. In the European population, CRC has an incidence of 31.3 new cases per 100,000 inhabitants, and this cancer generated in the EU 345,346 new cases and 152,046 deaths in 2012. These data make CRC the most common type of cancer in European population, as well as in Western countries. In addition, CRC is increasing every year in Western countries due to environmental risk factors (tobacco, alcohol, chlorine in water) and dietary habits (saturated fats, salty food nitrosamines, benzopyrenes from overcooked food, low consumption of fruit and vegetable fiber), which affect the colon mucosa healthy status.

Colon mucosa is structured as a monolayer epithelium (colonocytes) with millions of crypts. These crypts are invaginations which allow this tissue to increase the mucosal functional surface for absorption of nutrients. Also, these crypts contain, at their bottom, the stem cells in charge of renewal of the whole colon mucosa, which are cells with constant multiplication capabilities. These stem cells at the bottom of each colon mucosa crypt may suffer DNA mutations in genes (as *apc, k-ras, dcc* and *p53*)*,* leading to their transformation in cancer cells with uncontrolled growth, which will proliferate towards an aberrant crypt foci, then towards a microadenoma, towards a polyp (large adenoma), and finally towards a metastatic colon carcinoma.

The described tumorigenic process (starting from the initial aberrant crypt foci transformed cells in the colon mucosa) can be altered, or even stopped, by the presence in the colon lumen of some nutraceutical compounds as for example the prebiotic fibers. These prebiotic fibers, as inulin, are plant polysaccharide polymers formed by long D-fructose chains, which are not digested by human mouth, stomach, pancreatic nor intestinal enzymes. This is in contrast to other more abundant D-glucose plant polymers such as starch, which is fully digested towards free glucose and absorbed in the small intestine.

With respect to CRC prevention, these prebiotic fibers are very important, as once consumed, they are not digested nor absorbed in human digestive tract, arriving intact to the colon, where they selectively stimulate the growth or activity of some colon indigenous beneficial bacteria as *Lactobacillus, Bifidobacteria, Roseburia, Faecalibacterium* and other genera (Gibson et Roberfroid, 1995; Roberfroid, 2007; Pompei et al., 2008; Bosscher et al., 2009).

The colon microbiota fermentation of prebiotic fibers generates a type of compounds called short-chain fatty acids (SCFAs) as lactate, acetate, propionate, butyrate, isobutyrate, valerate, hexanoate, etc. (Rumessen et al., 1990). From these SCFAs, the most important one in colon homeostasis is butyrate, as normal colonocytes metabolize it in order to generate energy.

Polyhydroxyalkanoate polymers (PHAs), and particularly polyhydroxybutyrate (PHB), are bacterial energy reserves. These polymers lack toxicity in humans and are used for surgery and other medical applications. For example, nanoparticles made with PHB have been also used for diagnostic purposes on colon cancer (Kwon et al., 2014) and for delivery of conjugated antitumor proteins to HeLa uterus cancer cells in *in vitro* experiments (Pandian et al., 2015).

WO 00/04895 (Metabolix Inc.) relates to nutritional and therapeutic uses of 3-hydroxyalkanoate oligomers for increasing ketone body levels in the blood of humans and other mammals, which are suitable for oral or parenteral administration. Such oligomers provide a source of ketone bodies in the form of linear or cyclic oligomers and/or derivatives of 3-hydoxyacids. Representative methods for preparing the hydroxyacid oligomer derivatives include direct degradation of PHAs to oligomeric derivatives; polymerization of hydroxyalkanoates or derivatives thereof; and, stepwise synthesis of hydroxyalkanoate oligomers beginning or ending with modification of a terminal hydroxyalkanoate unit. The cyclic oligomers would have advantageous properties, since they would result in a sustained and/or controlled ketone blood level over a period of hours. Increasing blood ketone levels would be useful for seizure control, metabolic disease control, reduction of protein catabolism, appetite suppression, parenteral nutrition, increasing cardiac efficiency, treatment of diabetes and insulin resistant states, and treatment of effects of neurodegenerative disorders and epilepsy. IONUT-CRISTIAN RADU ET AL: "Poly(HydroxyButyrate-co-HydroxyValerate) (PHBHV) Nanocarriers for Silymarin Release as Adjuvant Therapy in Colo-rectal Cancer", FRONTIERS IN PHARMACOLOGY, vol. 8, 2 August 2017, discloses the use of poly(hydroxybutyrate-co-hydroxyvalerate) (PHBHV) nanocarriers loaded with silymarin to treat colorectal cancer.

The Applicant has studied the effects of oral administration of PHAs to mammals, particularly to humans, to find possible beneficial effects in preventing or treating various diseases, including cancer. Within such research program, the Applicant has postulated the existence of possible beneficial effects of PHAs on the development of diseases of the gastrointestinal tract and has surprisingly found that oral administration of a PHA containing 3-hydroxybutyrate monomeric units, particularly of a PHB, has a remarkable effect in preventing CRC. This is the surprising result of a non-pharmacological preclinical trial approach, which has been explored for prevention of CRC via the oral administration of PHB in a rat model, which is illustrated in the following.

Therefore, according to a first aspect, the present invention relates to a polyhydroxyalkanoate (PHA) containing 3-hydroxybutyrate monomeric units for use in the prevention of colorectal cancer (CRC) by oral administration. Preferably, the PHA is a polyhydroxybutyrate (PHB) homopolymer or a copolymer containing at least 20% by mole of 3-hydroxybutyrate monomeric units, the remaining being hydroxyalkanoate monomeric units different from 3-hydroxybutyrate.

Within the present description and the appended claims, the term "prevention" includes inhibition of initiation or of development of CRC, so as to avoid or at least substantially delay the onset of the CRC.

The oral administration may be an enteral administration to a subject, e.g. to a subject that has a predisposition in developing CRC or has already shown at least one clinical symptom which is typical of an early stage of CRC.

Alternatively, the oral administration may be a dietary administration, which can be directed to a vast majority of population, which wishes to obtain a preventive effect against the onset of CRC.

Without being bound to any specific biological mechanism that may be at the origin of such preventive effect on CRC, the Applicant believes that a PHA as such can be metabolized in the gastrointestinal tract to form 3-OH-butyrate, a short-chain fatty acid (SCFA) which can be eventually converted in other SCFAs, which are active as preventive agents in CRC, where they induce apoptosis (cell death) in colon tumor cells, whereas they act as the main nutrient in normal colon cells.

A PHA as such is a PHA obtained from the fermentation of an organic substrate by a PHA-producing microorganism, without any chemical modifications of the PHA structure, such as those described in WO 00/04895 cited above to obtain low molecular weight oligomers. The PHA may be subjected only to a purification phase, which is aimed to eliminate side products which may be present in PHAs and be unsuitable for oral administration, such as surfactants and cell residues. After purification, the PHA has usually a purity degree of at least 99.5%.

The PHAs suitable for the present invention are produced by microorganisms isolated from natural environments or also by genetically modified microorganisms, which act as carbon and energy reserves and which are accumulated by various species of bacteria under unfavorable growth conditions and in the presence of an excess carbon source. PHAs may be synthesized and accumulated by about 300 different microbial species, included within more than 90 kinds of Gram-positive and Gram-negative bacteria, such as, for example, *Bacillus, Rhodococcus, Rhodospirillum, Pseudomonas, Alcaligenes, Azotobacter, Rhizobium.* In cells, PHAs are stored in the form of microgranules, whose size and number per cell varies in the different bacterial species.

In general, the PHAs suitable for the present invention are homopolymers made of 3-hydroxybutyrate monomeric units:

-O-CH(CH₃)-CH₂-CO- (I)

or copolymers of 3-hydroxybutyrate monomeric units with at least one hydroxyalkanoate monomeric unit having formula

-O-CHR₁-(CH₂)ₙ-CO- (II)

wherein:
R₁ is selected from: -H and C₁-C₁₂ alkyls;
n is zero or an integer ranging from 1 to 6, and is preferably 1 or 2; with the proviso that, when R₁ is methyl, n is different from 1. Preferably, R₁ is methyl or ethyl. Preferably, n is 1 or 2.

In the case of a copolymer, it preferably contains at least 20% by mole, more preferably at least 30% by mole, of 3-hydroxybutyrate monomeric units, the remaining being hydroxyalkanoate monomeric units different from 3-hydroxybutyrate. In such copolymers, the amount of 3-hydroxybutyrate monomeric units is generally equal to or lower than 99% by mole, preferably equal to or lower than 90% by mole.

Particularly preferred repetitive units having formula (II) are those deriving from: 4-hydroxybutyrate, 3-hydroxyvalerate, 3-hydroxyhexanoate, 3-hydroxyoctanoate, 3-hydroxyundec-10-enoate, 4-hydroxyvalerate.

In the case of PHA copolymers, they are preferably selected from: poly(3-hydroxybutyrate-co-3-hydroxyvalerate) (PHBV), poly(3-hydroxybutyrate-co-3-hydroxyhexanoate) (PHBH), poly(3-hydroxybutyrate-co-4-hydroxybutyrate), poly(3-hydroxybutyrate-co-3-hydroxyvalerate-co-4-hydroxyvalerate (PHBVV), or mixtures thereof.

PHAs suitable for the present invention preferably have a weight average molecular weight (M_{w}) ranging from 5,000 to 1,500,000 Da, more preferably from 100,000 to 1,000,000 Da. The weight average molecular weight can be determined according to known techniques, in particular by means of GPC (Gel Permeation Chromatography) analysis.

As far as the production of PHAs is concerned, this is preferably obtained by microbial fermentation of an organic substrate (for example, carbohydrates or other fermentable substrates, such as glycerol) by means of a strain of microorganisms capable of producing PHAs, and the subsequent recovery of the PHAs from the cell mass. For further details, reference should be made, for example, to patent applications WO 99/23146, WO 2011/045625 and WO 2015/015315. Substrates suitable for the production of PHAs via fermentation can be obtained in particular from the processing of vegetables, for example juices, molasses, pulp from sugar beet processing, sugar cane. These substrates generally contain, in addition to sucrose and other carbohydrates, organic growth factors, nitrogen, phosphorous and/or other minerals useful as nutrients for cell growth. An alternative consists of glycerol, a low-cost organic carbon source, as it is a by-product of the production of biodiesel (see for example patent US 8 956 835 B2).

Since the PHAs suitable for the present invention are not water-soluble, they can be administered in the form of aqueous suspensions, wherein the PHA is in the form of suspended particles, preferably having an average size ranging from 0.1 µm to 1,000 µm, more preferably from 1 µm to 500 µm. These dimensions can be determined according to techniques well known in the art, such as particle-size detection systems in suspension with laser detectors, known as Dynamic Light Scattering (DLS) techniques (see the standard ISO 13320-2009). As an alternative, electron microscope images (SEM) can be used, which are digitally analyzed according to well known techniques.

Alternatively, the PHA can be administered as a functional component in different pharmaceutical products, where PHA particles may be suspended. Some examples are gel sachets, capsules, powder sachets and pills.

When administered as a dietary supplement, the PHA can be added to other edible products, such as functional drinks, fruit jellies, powder for resuspension in any liquid food matrix, cereal bars, powdered formula for breakfast, cookies, jelly beans, bubble gum, chocolate, fermented milk, ice cream, potato chips, bread or pasta.

As regards the dosage of the PHA for achieving a substantial effect in the prevention of CRC, of course it depends from the conditions of the subject and from the presence of external factors that may increase the risk of CRC development. Usually, the daily dosage may be generally equal to or higher than 0.3 g/kg body weight, preferably equal to or higher than 0.5 g/kg body weight. As regards an upper limit in the dosage, a daily dosage equal to or lower than 5.0 g/kg body weight, preferably equal to or lower than 2.5 g/kg body weight, is advisable.

The following examples are provided for purely illustrative purposes of the present invention and should not be considered as limiting the protection scope defined by the enclosed claims.

### 1. Animals and experimental design.

30 male rats of the strain *Rattus norvegicus* F344 were used for the following experiments to show the effect of administration of PHB as nutritional supplement on the prevention of CRC. This rat strain is commonly used as preclinical model for testing bioactive compounds against cancer, before entering in human clinical trials. In this mammal animal model, CRC is induced with two intraperitoneal injections of the carcinogenic compound azoxymethane, plus two oral doses of the pro-inflammatory dextran sodium sulfate. Azoxymethane is a carcinogenic compound that induces the formation of tumors in the colon. Dextran sodium sulfate is a pro-inflammatory compound that enhances the generation of tumors in the colon of these treated animals.

Rats (5 weeks old) were divided into 3 cohorts of 10 individuals each one, fed *ad libitum.*

Cohort 1 was fed with vegetable rat feed. This feed contained 16.7% protein, 5.8% fat and 53.6% carbohydrates (including 20% cellulose). This feed has a caloric value of 3.33 kcal/g.

Cohort 2 was fed with a version of this vegetable rat feed, but containing 10% PHB: 16.7% protein, 5.8% fat and 53.6% carbohydrates (including 10% cellulose). This feed has a caloric value of 3.88 kcal/g.

Cohort 3 was fed with a version of this vegetable rat feed, but containing 20% PHB: 16.7% protein, 5.8% fat and 53.6% carbohydrates (including 0% cellulose). This feed has a caloric value of 4.44 kcal/g.

The compositions of the different types of feed with respect to macronutrients and caloric value (both for each 20 g of feed as an average daily dose per rat, and as a percentage) are reported in Table 1.

**TABLE 1**

| | | Content in 20 g of feed | kcal in 20 g of feed | % kcal |
|---|---|---|---|---|
| Cohort 1 feed (control) | Carbohydrates | 10.72 | 42.62 | 64 |
| | Lipids | 1.16 | 10.65 | 16 |
| | Proteins | 3.34 | 13.32 | 20 |
| | PHB | 0 | 0 | 0 |
| | Total cal | - | 66.59 | - |
| Cohort 2 feed (10% PHB) | Carbohydrates | 10.72 | 42.62 | 54.92 |
| | Lipids | 1.16 | 10.65 | 13.72 |
| | Proteins | 3.34 | 13.32 | 17.16 |
| | PHB | 2 | 11.12 | 14.33 |
| | Total cal | - | 77.71 | - |
| Cohort 3 feed (20% PHB) | Carbohydrates | 10.72 | 42.62 | 47.99 |
| | Lipids | 1.16 | 10.65 | 11.99 |
| | Proteins | 3.34 | 13.32 | 15.00 |
| | PHB | 4 | 22.24 | 25.04 |
| | Total cal | - | 88.83 | - |

PHB has a caloric value of 5.56 kcal/g. Therefore, Table 1 shows the nutrients content and the daily caloric intake corresponding to 20 g of feed, which is the average value of ingested feed for an adult rat.

Animals in the control feed cohort 1 ingested 0 g of PHB per rat/day, animals in cohort 2 ingested 2 g of PHB per rat/day, and animals in cohort 3 ingested 4 g of PHB per rat/day. In total, along the 17 weeks nutritional intervention (119 days in total), each rat ingested an average of 0 g of PHB (cohort 1), 238 g of PHB (cohort 2) or 476 g of PHB (cohort 3).

### 2. CRC induction and monitoring.

One week after arrival of the animals to the animal facility, the three corresponding diets started. After one week feeding on the corresponding diet, CRC was induced in eight rats from each cohort. The two other rats were kept free of CRC induction, as absolute control animals. CRC induction was carried out in those eight rats of each cohort using azoxymethane (AOM, Sigma-Aldrich) dissolved in sterile saline (0.9% NaCl) at a concentration of 2 mg/mL. This AOM solution was injected intraperitoneally at a final concentration of 10 mg per kg body weight. The AOM treatment was repeated 7 days after the first injection. Absolute control animals received sterile saline in both injections.

One week after the second AOM treatment, those eight rats received drinking water during 7 days containing 3% dextran sodium sulfate (DSS, 40.000 g/Mol, VWR). This ulcerative colitis step was repeated after 11 weeks, using in this case 2% DSS during another 7 days.

Rats were sacrificed by pneumothorax on the 17th week, counted after the first administration of AOM. Rats were monitored along the whole experiment for body weight and stool consistency/rectal bleeding. Rats were weighted regularly during the experimental weeks.

### 3. Blood and tissue samples.

At week 17 after the first administration of AOM, rats were anesthetized (isoflurane) and sacrificed (pneumothorax) for extractions of blood (2 mL from heart, centrifuged at 3,000 rpm for 15 min, and then plasma was frozen), colon (opened longitudinally and washed with PBS before keeping it in 4% formaldehyde at 4°C) and caecum (frozen at -20°C). Caecums were weighed right after sacrifice using a precision scale.

Fixed colons were meticulously examined with a precision gauge for counting the number of tumors bigger than 1 mm on its inner mucosa surface. Moreover, the total tumor affected area was calculated, after classification of the tumors shape in pedunculated, plane irregular, plane circular and spherical.

### 4. Statistical methods.

Normality of the different variables was tested using Shapiro-Wilk's test. In light of these results, data were expressed then as the mean value ± S.E.M. and parametric methods were used for statistical analyses. Equality of variances was tested using Levene's test and then the differences among cohorts were tested by a 1-way ANOVA analysis of variance.

The graphical representation of all these data was carried out using GraphPad Prism software, version 7. In all cases, a value of p<0.05 was considered statistically highly significant (*: p<0.05; **: p<0.005; ***: p<0.0005; ****: p<0.0001).

### 5. Results.

### 5.1 Evolution of body weight gain.

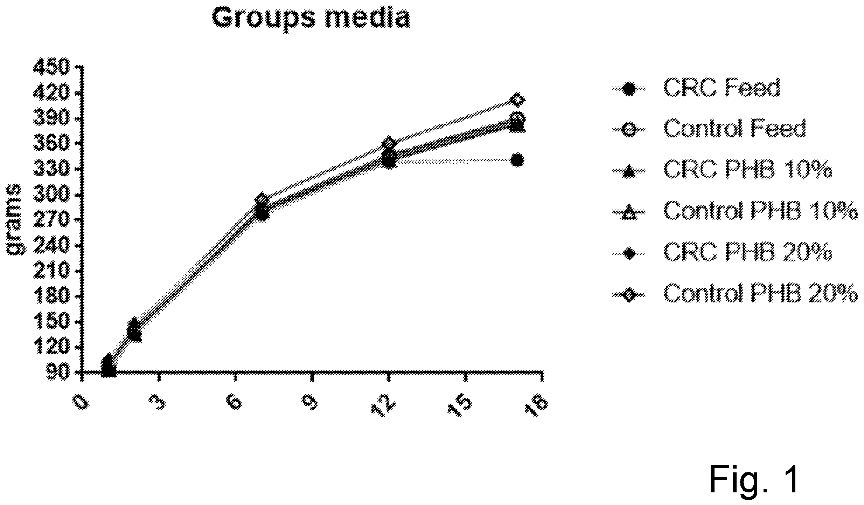

During all the experimental timeframe, all rats were weighted on a regular basis, in order to test if some of the diet treatments were showing toxicity to the rats, which could be eventually observed as weight loss. In all cases, none of the three different diet types has generated weight changes of interest (see Figure 1). Absolute control animals (rats number 9 and 10) from cohort 3 showed a little increased body weight as average, but these differences were not statistically highly significant (p = 0.05).

### 5.2 Evolution of digestive symptoms.

Each rat cohort contained two absolute control rats (animals 9 and 10 in each case). These two animals were those ones feeding on the corresponding diet (control, 10% PHB or 20% PHB), but getting only intraperitoneal PBS solution (instead of the carcinogen compound AOM). Moreover, they never got the pro-inflammatory agent DSS in the drinking water. Therefore, these two animals in each cohort are supposed to lack any type of digestive symptom, and actually they have not shown any digestive symptom until sacrifice time. This means that PHB ingestion is not toxic to animals at those doses (10% and 20%), at least with respect to digestive symptoms. Moreover, the weight gain was similar between the three types of absolute control animals (see below).

With respect to the experimental animals, those ones where CRC was induced with AOM and DSS treatments, some symptoms were observed in the three diet cohorts (control, 10% PHB and 20% PHB). Specifically, some of these experimental rats have shown diarrhea or gastrointestinal hemorrhages during both DSS challenges. These hemorrhagic symptoms were classified as follows:
- mild diarrhea without presence of blood;
- diarrhea with the presence of blood drops in feces;
- diarrhea with mild hemorrhage in feces.

Table 2 summarizes the situation of these gastrointestinal side effects for the 30 rats, including animals 9 and 10 (absolute control animals, without CRC).

**TABLE 2**

| | Control feed (cohort 1) | | 10% PHB feed (cohort 2) | | 20% PHB feed (cohort 3) | |
|---|---|---|---|---|---|---|
| Rats numbers | 1 to 8 (a) | 9, 10 (b) | 1 to 8 (a) | 9, 10 (b) | 1 to 8 (a) | 9, 10 (b) |
| No symptoms | 2, 8 | 9, 10 | 1, 2, 5 | 9, 10 | 1, 3, 4, 5, 8 | 9, 10 |
| Mild diarrhea | - | - | - | - | 2, 6 | - |
| Diarrhea with blood drops | 1, 4, 6, 7 | - | 3, 6, 8 | - | 7 | - |
| Diarrhea with hemorrhage | 3 (†), 5 | - | 4, 7 | - | - | - |

| | | | | | | |
|---|---|---|---|---|---|---|
| (a) tumors induction (b) absolute controls (†) dead | | | | | | |

As can be observed from Table 2, an animal (rat number 3) was dead during the experiment in control feed group. This rat was showing diarrhea with blood drops during the second DSS challenge. DSS treatments induce a pro-inflammatory status at the colon mucosa with bleeding, and this is necessary to induce tumor formation. Moreover, after the first DSS challenge another rat from control feed group (rat number 5) showed bleeding (see Table 2).

The fact that no deaths took place in cohorts 2 and 3 indicated that these rats resisted better the tumor induction and the DSS pro-inflammatory challenges.

In summary, severity of the gastrointestinal side effects after the DSS treatments was more intense in the control feed cohort. Rats showing mild diarrhea only existed in cohort 3 (2 rats) . The number of rats showing diarrhea with blood drops was higher in control feed cohort 1 (4 rats) than in cohort 2 (3 rats) or cohort 3 (1 rat) (see Table 2). Finally, there were no rats showing diarrhea with hemorrhage in cohort 3, but 2 rats of this type were present in cohort 2 and control feed cohort 1 (one of these two rats from the control feed cohort died).

### 5.3 Caecum weight.

Caecum is the section of the digestive tract in charge of the digestion of prebiotic fibers, due to the presence in this organ of the corresponding bacterial populations with this capacity.

Therefore, it was expected that the presence of a putative prebiotic compound, in this case PHB, could enhance microbial populations in caecums, giving rise to organs with increased microbial biomass in the cases of cohorts 2 and 3.

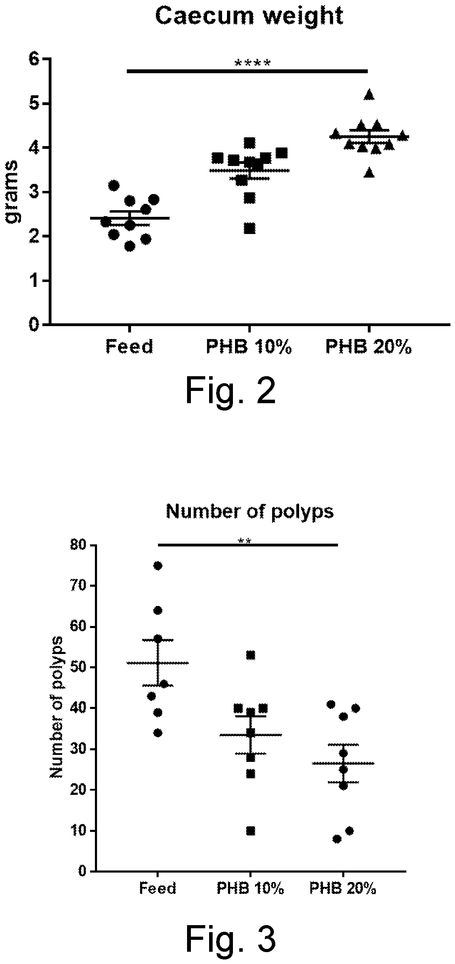

After sacrificing the animals, the caecums were weighted immediately. Statistically highly significant differences for these values were observed among the control feed and cohort 3 (p value less than 0.0001) (Figure 2). There was also an increase in caecum weight in the case of cohort 2 with respect to control feed cohort 1.

The results are reported in Figure 2, a diagram showing the caecum weight measured in the three animal cohorts along the experiment. Mean and SEM values are indicated for each animal cohort with horizontal lines (control feed cohort 1: 2.414 g ± 0.15; cohort 2: 3.488 g ± 0.18; cohort 3: 4.252 g ± 0.14). These data indicate that the addition of PHB to the diet exerts an effect in caecum microbial populations, which are enhanced as in the case of a supplementation with a conventional prebiotic fiber.

### 5.4 Number and total area of colorectal tumors.

After the sacrifices of the rats, colon mucosas were analyzed, and tumors bigger than 1 mm were quantified. Figure 3 shows the average colon tumors number from each cohort (rats 1 to 8 in the three cases). Absolute control animals (rats 9 and 10 from each cohort) showed not tumors, as expected. The values are: control feed cohort 1: 51.14 tumors ± 5.57; cohort 2: 33.5 tumors ± 4.56; cohort 3: 26.5 tumors ± 4.58.

A statistical highly significant difference (p = 0.0058) was particularly observed among control feed cohort 1 and cohort 3, which showed a drastic 48.18% reduction in the number of colon tumors (see Figure 3). The number of tumors was also reduced (34.49% reduction) in the case of cohort 2 with respect to control feed cohort 1 (see Figure 3).

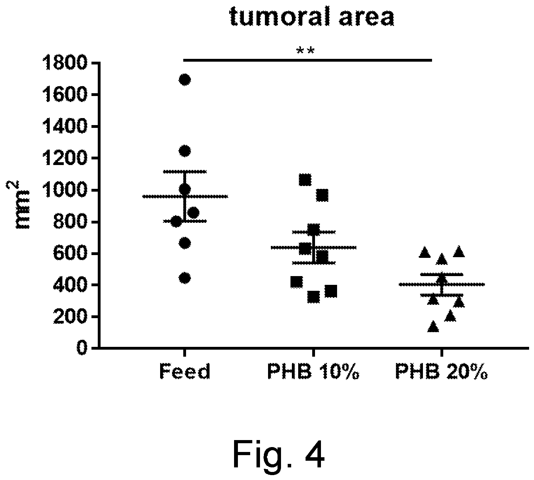

Moreover, depending on the shape of each tumor, the tumor area was calculated for each of the existing tumors in a given colon mucosa, and the total value of tumor area was generated for each animal. Figure 4 shows the average value, in mm², of the total tumor affected area in the colons from each cohort. The values are: control feed cohort 1: 960.1 mm² ± 155.1; cohort 2: 638.2 mm² ± 97.17; 20% cohort 3: 402.2 mm² ± 65.67.

Cohort 3 showed a 58.1% reduction in this parameter with respect to control feed cohort 1, and reduction was statistically highly significant (0.0047) (see Figure 4). The total tumor area was also reduced (35.52% reduction) in the case of cohort 2 with respect to control feed cohort 1.

In conclusion, addition of 10% PHB or 20% PHB to the diet of animals in this experimental murine model for CRC cancer showed an important prevention against the development of colorectal tumors. The absence of toxicity in PHB could allow its use as a preventive agent for this type of digestive cancer in humans.

### Bibliography

Gibson GR, Roberfroid MB, Dietary modulation of the human colonic microbiota: introducing the concept of prebiotics; J Nutr 1995; 125:1401-1412.
Roberfroid MB, Inulin-type fructans: functional food ingredients; J Nutr 2007; 137:2493S-2502S.
Pompei A, Cordisco L, Raimondi S, Amaretti A, Pagnoni UM, Matteuzzi D, Rossi M, In vitro comparison of the prebiotic effects of two inulin-type fructans Anaerobe; 2008 Nov; 14(5):280-6.
Bosscher D, Breynaert A, Pieters L, Hermans N, Food-based strategies to modulate the composition of the intestinal microbiota and their associated health effects; J Physiol Pharmacol. 2009 Dec; 60 Suppl 6:5-11.
Rumessen JJ, Bode S, Hamberg O, Gudmand-31. Hoyer E, Fructans of Jerusalem artichokes: intestinal transport, absorption, fermentation, and influence on blood glucose, insulin, and C-peptide responses in healthy subjects; Am J Clin Nutr 1990; 52:675-681.
Kwon HS, Jung SG, Kim HY, Parker SA, Batt CA, Kim YR, A multi-functional polyhydroxybutyrate nanoparticle for theranostic applications; J. Mater. Chem. B, 2014, 2, 3965-3971.
Pandian SR, Deepak V, Nellaiah H, Sundar K, PEG-PHB-glutaminase nanoparticle inhibits cancer cell proliferation in vitro through glutamine deprivation; In Vitro Cell Dev Biol Anim. 2015; 51(4):372-80.

## Claims

1. A polyhydroxyalkanoate (PHA) containing 3-hydroxybutyrate monomeric units for use in the prevention of colorectal cancer (CRC) by oral administration.

2. The PHA for use according to claim 1, wherein the PHA is a polyhydroxybutyrate (PHB) homopolymer or a copolymer containing at least 20% by mole of 3-hydroxybutyrate monomeric units, the remaining being hydroxyalkanoate monomeric units different from 3-hydroxybutyrate.

3. The PHA for use according to claim 2, wherein the PHA is poly-3-hydroxybutyrate (PHB).

4. The PHA for use according to claim 2, wherein the hydroxyalkanoate monomeric units different from 3-hydroxybutyrate are selected from: 4-hydroxybutyrate, 3-hydroxyvalerate, 3-hydroxyhexanoate, 3-hydroxyoctanoate, 3-hydroxyundec-10-enoate, 4-hydroxyvalerate.

5. The PHA for use according to anyone of the preceding claims, wherein the PHA has a weight average molecular weight (M_{w}) ranging from 5,000 to 1,500,000 Da, preferably from 100,000 to 1,000,000 Da.

6. The PHA for use according to anyone of the preceding claims, wherein the oral administration is an enteral administration to a subject that has a predisposition in developing CRC or has already shown at least one clinical symptom which is typical of an early stage of CRC.

7. The PHA for use according to anyone of the preceding claims, wherein the oral administration is a dietary administration.

8. The PHA for use according to anyone of the preceding claims, wherein the oral administration has a daily dosage equal to or higher than 0.3 g/kg body weight, preferably equal to or higher than 0.5 g/kg body weight.

9. The PHA for use according to anyone of the preceding claims, wherein the oral administration has a daily dosage equal to or lower than 5.0 g/kg body weight, preferably equal to or lower than 2.5 g/kg body weight.

## Patentansprüche

1. Polyhydroxyalkanoat (PHA), das 3-Hydroxybutyrat-Monomereinheiten enthält, zur Verwendung bei der Prävention von kolorektalem Krebs (CRC) durch orale Verabreichung.

2. PHA zur Verwendung nach Anspruch 1, wobei das PHA ein Polyhydroxybutyrat (PHB)-Homopolymer oder ein Copolymer ist, das mindestens 20 Mol-% 3-Hydroxybutyrat-Monomereinheiten enthält, wobei der Rest Hydroxyalkanoat-Monomereinheiten sind, die sich von 3-Hydroxybutyrat unterscheiden.

3. PHA zur Verwendung nach Anspruch 2, wobei das PHA ein Poly-3-Hydroxybutyrat (PHB) ist.

4. PHA zur Verwendung nach Anspruch 2, wobei die von 3-Hydroxybutyrat verschiedenen Hydroxyalkanoat-Monomereinheiten ausgewählt sind aus: 4-Hydroxybutyrat, 3-Hydroxyvalerat, 3-Hydroxyhexanoat, 3-Hydroxyoctanoat, 3-Hydroxyundec-10-enoat, 4-Hydroxyvalerat.

5. PHA zur Verwendung nach einem der vorhergehenden Ansprüche, wobei das PHA ein gewichtsmittleres Molekulargewicht (M_{w}) im Bereich von 5.000 bis 1.500.000 Da, vorzugsweise von 100.000 bis 1.000.000 Da aufweist.

6. PHA zur Verwendung nach einem der vorhergehenden Ansprüche, wobei die orale Verabreichung eine enterale Verabreichung an eine Person ist, die eine Prädisposition für die Entwicklung von CRC hat oder bereits mindestens ein klinisches Symptom gezeigt hat, das für ein frühes Stadium von CRC typisch ist.

7. PHA zur Verwendung nach einem der vorhergehenden Ansprüche, wobei die orale Verabreichung eine diätetische Verabreichung ist.

8. PHA zur Verwendung nach einem der vorhergehenden Ansprüche, wobei die orale Verabreichung eine Tagesdosis gleich oder höher als 0,3 g/kg Körpergewicht, vorzugsweise gleich oder höher als 0,5 g/kg Körpergewicht aufweist.

9. PHA zur Verwendung nach einem der vorhergehenden Ansprüche, wobei die orale Verabreichung eine Tagesdosis gleich oder niedriger als 5,0 g/kg Körpergewicht, vorzugsweise gleich oder niedriger als 2,5 g/kg Körpergewicht aufweist.

## Revendications

1. Polyhydroxyalcanoate (PHA) contenant des unités monomères de 3-hydroxybutyrate pour une utilisation dans la prévention du cancer colorectal (CRC) par administration orale.

2. PHA pour une utilisation selon la revendication 1, dans lequel le PHA est un homopolymère de polyhydroxybutyrate (PHB) ou un copolymère contenant au moins 20 % en mole d'unités monomères de 3-hydroxybutyrate, le reste étant des unités monomères d'hydroxyalcanoate différentes du 3-hydroxybutyrate.

3. PHA pour une utilisation selon la revendication 2, dans lequel le PHA est le poly-3-hydroxybutyrate (PHB) .

4. PHA pour une utilisation selon la revendication 2, dans lequel les unités monomères d'hydroxyalcanoate différentes du 3-hydroxybutyrate sont sélectionnées parmi : le 4-hydroxybutyrate, le 3-hydroxyvalérate, le 3-hydroxyhexanoate, le 3-hydroxyoctanoate, le 3-hydroxyundec-10-énoate, le 4-hydroxyvalérate.

5. PHA pour une utilisation selon l'une quelconque des revendications précédentes, dans lequel le PHA possède un poids moléculaire moyen en poids (M_{w}) allant de 5 000 à 1 500 000 Da, de préférence de 100 000 à 1 000 000 Da.

6. PHA pour une utilisation selon l'une quelconque des revendications précédentes, dans lequel l'administration orale est une administration entérale chez un sujet qui présente une prédisposition pour le développement d'un CRC ou qui a déjà présenté au moins un symptôme clinique qui est typique d'un stade précoce d'un CRC.

7. PHA pour une utilisation selon l'une quelconque des revendications précédentes, dans lequel l'administration orale est une administration alimentaire.

8. PHA pour une utilisation selon l'une quelconque des revendications précédentes, dans lequel l'administration orale présente un dosage quotidien égal ou supérieur à 0,3 g/kg de poids corporel, de préférence égal ou supérieur à 0,5 g/kg de poids corporel.

9. PHA pour une utilisation selon l'une quelconque des revendications précédentes, dans lequel l'administration orale présente un dosage quotidien égal ou inférieur à 5,0 g/kg de poids corporel, de préférence égal ou inférieur à 2,5 g/kg de poids corporel.
